# EUROPEAN PATENT APPLICATION

(11) **EP 0 651 249 A2**
(43) Date of publication of application: **03.05.1995**
(21) Application number: 94308091.1
(22) Date of filing: 02.11.1994
(51) Int. Cl.: G01N 33/487, G01N 33/49, G01N 33/493, G01N 33/50, G01N 33/53

(54) **Analytical device**

(30) Priority: 03.11.1993 GB 9322650
(71) Applicant: COGENT DIAGNOSTICS LIMITED, Edinburgh EH9 3JF (GB)
(72) Inventor: Gordon, Michael John, Edinburgh, EH8 7JG (GB); Weston, James, Haddington, East Lothian, EH41 3NN (GB)
(74) Representative: Armitage, Ian Michael

(57) **Abstract**

An analytical device 2 for detecting analytes in a liquid sample is disclosed having a housing containing an assay membrane 12. The flow of liquids across and through the membrane 12 can be controlled by providing a relatively movable barrier 14 which in one configuration tends to block the flow of liquid through the membrane 12, thereby directing liquid across the membrane, while relative displacement of the barrier to a second configuration allows liquid to pass through the membrane, eg for washing steps. Additionally or alternatively, the membrane 12 can be movable into and out of contact with absorbent material 18 so that in one configuration in which the membrane 12 is in contact with the absorbent material 18, liquids tend to be drawn through the membrane, while in a second configuration in which the membrane is held away from the absorbent material, liquids tend to flow across the membrane.

## Description

The present invention relates to an analytical device.

In the past, many diagnostic assays for analytes in liquid samples have been carried out using dipsticks having binding agents, eg antibodies specific for the analytes in the sample, immobilised at discrete locations on their surfaces. In use, such dipsticks are sequentially contacted with liquid samples containing analyte and assay reagents.

However, the flow of the liquid sample and assay reagents across the surface of such dipsticks is uncontrolled, with the result that contact between the binding agent and the liquid sample or reagents can be inefficient. This in turn can lead to errors in detecting the analytes in the sample.

Furthermore, the scale of some diseases, eg rheumatic disease which effects approximately 7 million people in the UK alone, means that there is a continuing need to develop cost effective and accurate assay devices to assist in the diagnosis of these diseases.

Broadly, the present invention provides an analytical device in which the flow of liquids across and through an assay membrane can be controlled. This can be achieved by providing a relatively movable barrier which in one configuration tends to block the flow of liquid through the assay membrane, thereby directing liquid across the assay membrane, while relative displacement of the barrier to a second configuration allows liquid to pass through the assay membrane. Additionally or alternatively, the assay membrane can be movable into and out of contact with absorbent material so that in one configuration in which the membrane is in contact with the absorbent material, liquids tend to be drawn through the membrane, while in a second configuration in which the membrane is held away from the absorbent material, liquids tend to flow across the membrane.

Accordingly, in one embodiment, the present invention provides an analytical device for detecting analytes in a liquid sample comprising;
(a) a housing having a port for introducing liquids into the device;
(b) an assay membrane within the housing, disposed so as to be contactable by liquid passed in through the port, said membrane having reactive areas specific for analytes in the sample; and,
(c) a barrier,

wherein the barrier and assay membrane are movable relative to one another between a first position in which the barrier blocks the flow of the liquid through the assay membrane, thereby facilitating the flow of the liquid across the membrane, and a second position in which liquid can pass through the assay membrane.

In a second embodiment, the present invention provides an analytical device for detecting analytes in a liquid sample comprising;
(a) a housing having a port for introducing liquids into the device, the housing containing absorbent material;
(b) an assay membrane within the housing, disposed so as to be contactable by liquid passed in through the port, said membrane having reactive areas specific for analytes in the sample,

wherein the housing is divided into sections that are movable relative to each other, the movement causing the assay membrane to move into and out of contact with the absorbent material, so that in a first position in which the assay membrane is in contact with the absorbent material, liquid can pass through the membrane, while in a second position in which the membrane is not in contact with the absorbent material, the flow of liquid across the membrane is facilitated.

Preferably, the device can also store the excess liquid sample and reagents introduced into it after they have contacted the assay membrane. This has the advantage that the device encapsulates all the biological waste material used in the assay, allowing this waste material to be safely disposed of.

Preferably, the waste material is stored in absorbent material. This is preferably provided with a sintered membrane on the surface through which the waste can pass. The sintered membrane facilitates passage of liquids introduced into the device across the entire assay membrane, before they pass through the membrane to be stored in the absorbent material, which may act as a wick.

Typically, the liquid sample containing the analytes to be detected is whole blood, serum, plasma, saliva or urine. The use of saliva is preferred as the sample can be easily taken from patients. As the sample is forced to flow across the assay membrane, typically no dilution or separation of the liquid sample is required before introducing the sample into the device.

Preferably a reactive area on the assay membrane comprises a binding agent, eg an antibody, immobilised on the assay membrane in a discrete location, eg a spot. The present invention therefore allows for the assay of a plurality of analytes by immobilising a plurality of binding agents on the surface at discrete locations. Similarly, control locations can be included on the assay membrane for comparison with the reactive areas.

Conveniently, the housing of the assay device is cylindrical and is divided into cylindrical sections which are rotatable about the longitudinal axis of the device relative to each other. A port in the device allows sample and reagents to be introduced onto the assay surface.

In the first embodiment described above, the barrier and assay membrane are conveniently planar, part-circular surfaces (eg each being semicircular) and are mounted on adjacent sections, with the assay membrane held between the barrier and the port. Both the barrier and assay membrane are held approximately parallel to each other and to the flat faces of the cylinder. Typically, the barrier is made of a hydrophobic material.

Thus, in the first position the barrier lies adjacent and "eclipses" the assay membrane, preventing liquid sample passing through the assay membrane. In the second position, the sections on which the barrier and assay membrane are mounted are rotated relative to each other, with the result that the assay membrane and barrier are no longer in the eclipse relationship. This allows reagents to pass through the assay membrane without being blocked by the barrier.

In the second embodiment, adjacent sections of the housing contain the absorbent material and the assay membrane so that the rotation of the sections about the longitudinal axis of the device causes the membrane and absorbent material to move relative to each other, moving the assay membrane into or out of contact with the absorbent material.

In either embodiment, one of the sections can be conveniently provided with grooves and the other with lugs adapted to locate in the grooves so that when the sections are rotated, the lugs and grooves guide the movement of the sections. Thus, the membrane and the barrier or the membrane and absorbent material move relative to each other between the first and second positions as the sections are twisted by the user of the device. Additionally, the grooves can define the amount of rotation, eg 1/2 a turn, that is needed to change the device between configurations.

In the second embodiment, the grooves may be angled with respect to the longitudinal axis of the device so that the relative rotation of the sections causes the membrane to move into and out of contact with the absorbent material.

The present invention also includes a kit for carrying out an assay comprising an analytical device as described above, in combination with the reagents for carrying out the assay.

Embodiments of the present invention will now be described by way of example with reference to the accompanying drawings in which:
Figures 1 and 3 show schematic, vertical cross-sections through an analytical device according to the first embodiment of the present invention showing the device in two configurations;
Figures 2 and 4 show top plan views of the embodiment, in the configurations shown in figures 1 and 3;
Figure 5 shows an exploded, vertical cross-sectional view through the housing of an analytical device according to the second embodiment of the invention; and,
Figure 6 shows an exploded, vertical cross-sectional view through the housing of figure 5, showing absorbent material in place.

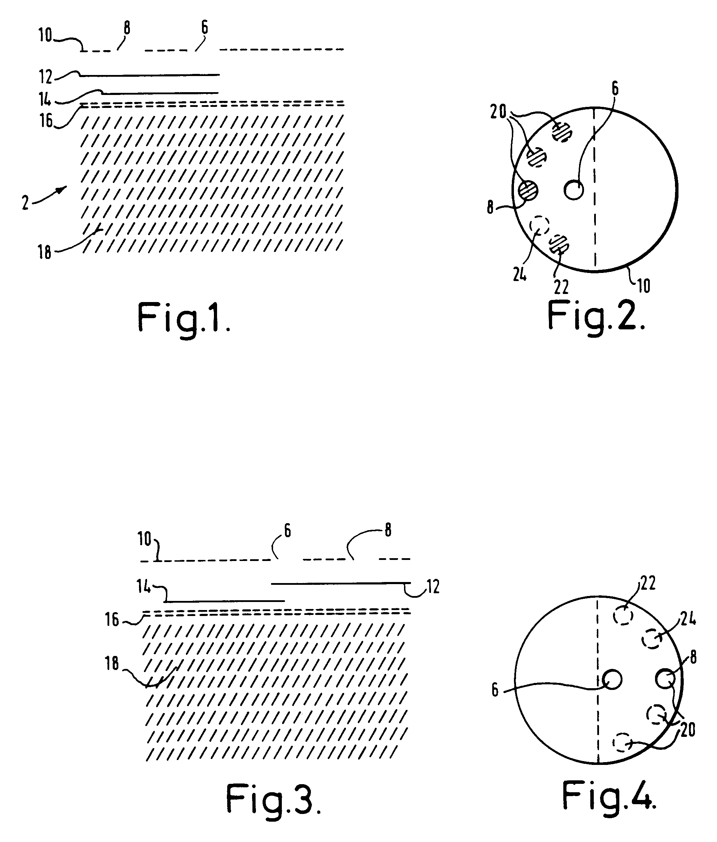

Figures 1 and 3 show an assay device 2 has a cylindrical housing which includes a round top cover portion 10 having a port 6 through which sample and reagents can be introduced and a second port or transparent portion 8 through which readings can be made. The assay device has a semicircular hydrophobic barrier 14 mounted between a semicircular assay membrane 12 and a circular sintered membrane 16. A matrix of absorbent material 18 under the sintered membrane 16 serves as a wick to draw liquid introduced into the device and may further serve to store waste reagents and sample.

The housing of the assay device 2 is divided into cylindrical sections with the assay membrane 12 and the hydrophobic barrier 14 mounted on adjacent sections. These sections are rotatable relative to one another about the longitudinal axis of the device. The assay membrane 12 and hydrophobic barrier 14 are planar, semi-circular surfaces, and in a first position shown in figure 1, the hydrophobic barrier 14 is held eclipsed between the assay membrane 12 and the sintered membrane 16.

However, rotating the sections relative to each other through 180° produces the configuration shown in figure 3 in which the assay membrane 12 and hydrophobic barrier 14 are not eclipsed. Therefore, liquid introduced into the device through port 6 can flow through the assay membrane 12 and through the sintered membrane 16 and is not blocked by the hydrophobic barrier 14.

Figures 2 and 4 show plan views of the device showing reactive areas 20 of binding agent, in this case different antibodies (IgA, IgG and IgM) to bind rheumatoid factors, immobilised on the assay membrane 12 in a series of discrete spots. Also immobilised on the assay membrane 12 are positive 22 and negative 24 controls. The reactive areas 20 and controls 22, 24 are arranged a common distance from the central axis of the device so that liquid sample and reagents introduced into the device flow an equal distance across the assay membrane 12 to reach them. The top portion 10 of the device can be incrementally rotated relative to the rest of the device, allowing the spots to be individually viewed through the second port 8 in order to take the results of the assay.

In use, the liquid sample is introduced into the device 2 through port 6 while it is in the position shown in figures 1 and 3. The liquid sample contacts the assay membrane 12 directly under port 6 and flows outwards towards the reactive areas 20 and controls 22, 24. If the liquid sample is whole blood, red blood cells adhere to the assay membrane around the area of contact, while plasma containing the analytes can flow outwards towards the reactive areas 20 and controls 22, 24. This avoids the need for separating the cells from the plasma. The liquid sample is prevented from passing through the assay membrane 12 by the hydrophobic barrier 14 eclipsed between the assay membrane 12 and the sintered membrane 16.

Once the sample has been applied to the assay membrane and has contacted the reactive areas 20 and controls 22, 24, the section of the device on which the assay membrane 12 is mounted is rotated into the position shown in figures 3 and 4. In this position, the hydrophobic barrier 14 is no longer eclipsed between the assay membrane 12 and the sintered membrane 16. Thus, when a liquid, for example a wash buffer, is introduced into the device through port 6, it is able to pass through the assay membrane 12, after contacting the reactive areas 20 and controls 22, 24. The sintered membrane 16 acts to draw reagent across the entire assay membrane 12, providing complete and even contact. The liquid is then drawn through the sintered membrane 16 into the wick 18 which serves as a reservoir to store the waste reagents.

Once washing is complete, the device 2 is rotated back to the position shown in figures 1 and 2. A conjugate can then be introduced into the device 2 and the device rotated into the position shown in figures 3 and 4 for further washing. The device 2 is then rotated back into the position shown in figures 1 and 2. Typically, substrates will then be added through port 6 to react with the analytes bound to the reactive areas 20 and produce a detectable result, eg a colour change. Alternatively, coloured latex particles having antibody immobilised their surfaces can be added after a single washing step to produce a detectable result.

When the colour has developed, the results can be read by rotating the top portion 10 of the device until the second port 8 is above the reactive area or control area to be read. This has the advantage that each reading is made separately, avoiding any confusion from adjacent reactive areas or controls.

Once the assay has been carried out, the analytical device 2 can be disposed of as a single unit encapsulating the sample and all the reagents used in the assay.

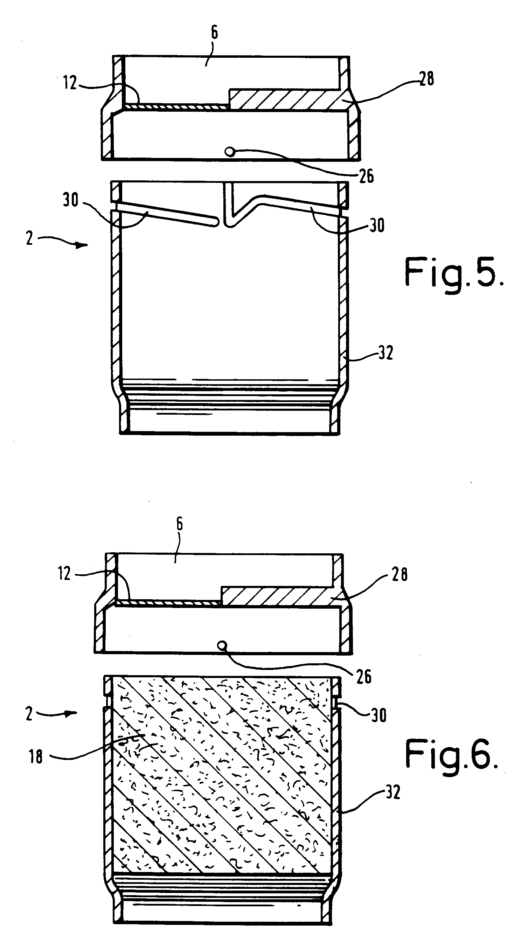

Figures 5 and 6 show an assay device 2 generally similar to the device of figures 1 to 4, except that it does not have a movable hydrophobic barrier to control the flow of liquids through and across the assay membrane 12. Thus, in the device of figures 5 and 6, the flow of liquid introduced through port 6 is controlled by bringing the assay membrane 12 into and out of contact with the absorbent wick 18. This is achieved by providing lugs 26 on the inside surface of the top section 28 of the device 2 on which the assay membrane 12 is carried and grooves 30 on the outer surface of the lower section 32 containing the absorbent material (wick) 18.

The grooves 30 are angled with respect to the longitudinal axis of the device and each groove runs around half of the circumference of the housing. Thus, in use the two sections 28, 32 can be rotated relative to each other through 180°, bringing the membrane 12 into or out of contact with the absorbent material 18, as required for carrying out an assay.

Thus, when the membrane 12 is in contact with the absorbent material 18, this facilitates the flow of liquid through the membrane, eg during washing, while when the membrane is held above the absorbent material, the flow of liquids across the membrane is encouraged.

The grooves 30 and lugs 26 also help to define the amount of rotation needed to change the device between configurations, making the device easy to use and helping to ensure that each assay is carried out under a fixed set of conditions.

## Claims

1. An analytical device for detecting analytes in a liquid sample comprising;
(a) a housing having a port for introducing liquids into the device;
(b) an assay membrane within the housing, disposed so as to be contactable by liquid passed in through the port, said membrane having reactive areas specific for analytes in the sample; and,
(c) a barrier,
wherein the barrier and assay membrane are movable relative to one another between a first position in which the barrier blocks the flow of the liquid through the assay membrane, thereby facilitating the flow of the liquid across the membrane, and a second position in which liquid can pass through the assay membrane.

2. An analytical device for detecting analytes in a liquid sample comprising;
(a) a housing having a port for introducing liquids into the device, the housing containing absorbent material;
(b) an assay membrane within the housing, disposed so as to be contactable by liquid passed in through the port, said membrane having reactive areas specific for analytes in the sample,
wherein the housing is divided into sections that are movable relative to each other, the movement causing the assay membrane to move into and out of contact with the absorbent material, so that in a first position in which the assay membrane is in contact with the absorbent material, liquid can pass through the membrane, while in a second position in which the membrane is not in contact with the absorbent material, the flow of liquid across the membrane is facilitated.

3. The analytical device according to claim 1 or claim 2 wherein the housing is divided into sections that are relatively rotatable about the longitudinal axis of the device, the rotation of the sections causing the barrier and membrane to move between said first and second positions.

4. The analytical device according to claim 2 wherein one of the sections is provided with grooves and the other with lugs adapted to locate in the grooves so that when the sections are rotated, the lugs and grooves guide the relative movement of the sections between the first and second positions.

5. The analytical device according to any one of the preceding claims wherein the device is capable of storing liquids passing through the membrane.

6. The analytical device according to claim 5 wherein the liquids are stored in absorbent material.

7. The analytical device according to claim 6 wherein the absorbent material has a sintered membrane on its surface to facilitate the passage of liquid into the absorbent material.

8. The analytical device according to any one of the preceding claims wherein the liquid sample containing the analytes is whole blood, serum, plasma, saliva or urine.

9. The analytical device according to any one of the preceding claims wherein the reactive area on the assay membrane comprises a binding agent specific for an analyte in the sample.

10. The analytical device according to any one of the preceding claims wherein the assay membrane includes control areas for comparison with the reactive areas.

11. A kit comprising an analytical device according to any one of the preceding claims, in combination with reagents for carrying out the assay.
